(19) Europäisches Patentamt
European Patent Office
Office européen des brevets

(11) **EP 2 547 376 B1**

(12) **EUROPÄISCHE PATENTSCHRIFT**

(45) Veröffentlichungstag und Bekanntmachung des Hinweises auf die Patenterteilung:
**06.05.2015 Patentblatt 2015/19**

(21) Anmeldenummer: **11710694.8**

(22) Anmeldetag: **17.03.2011**

(51) Int Cl.:
***A61M 1/10*** *(2006.01)* ***A61M 1/14*** *(2006.01)*

(86) Internationale Anmeldenummer:
**PCT/EP2011/001333**

(87) Internationale Veröffentlichungsnummer:
**WO 2011/113602 (22.09.2011 Gazette 2011/38)**

(54) **VORRICHTUNG ZUR REGELUNG WENIGSTENS EINES FILTRATIONSWERTES UND HÄMODIALYSEMASCHINE MIT EINER DERARTIGEN VORRICHTUNG**

DEVICE FOR REGULATING AT LEAST ONE FILTRATION VALUE AND HAEMODIALYSIS MACHINE WITH SUCH A DEVICE

DISPOSITIF DE RÉGULATION D'AU MOINS UNE VALEUR DE FILTRATION ET HÉMODIALYSEUR AVEC UN TEL DISPOSITIF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.03.2010 DE 102010012050**

(43) Veröffentlichungstag der Anmeldung:
**23.01.2013 Patentblatt 2013/04**

(73) Patentinhaber: **Fresenius Medical Care Deutschland GmbH**
**61352 Bad Homburg (DE)**

(72) Erfinder:
• **PETERS, Arne**
**61352 Bad Homburg (DE)**
• **HEIDE, Alexander**
**65817 Eppstein (DE)**
• **WIKTOR, Christoph**
**63571 Gelnhausen (DE)**

(74) Vertreter: **Herrmann, Uwe et al**
**Lorenz - Seidler - Gossel**
**Widenmayerstrasse 23**
**80538 München (DE)**

(56) Entgegenhaltungen:
**EP-A1- 0 967 475     EP-A2- 1 424 089**
**WO-A1-96/31934     WO-A1-2005/063320**
**WO-A2-2006/135934     US-A- 4 781 525**

**Beschreibung**

Die vorliegende Erfindung betrifft eine Vorrichtung zur Regelung eines Filtrationswertes in einer Maschine zur Behandlung einer medizinischen Flüssigkeit, insbesondere einer Blutbehandlungsmaschine sowie weiter insbesondere zur Verwendung in Zellseparatoren und bei der Apherese, mit wenigstens einem Zentrifugalpumpmittel, eine Hämodialysemaschine sowie ein Verfahren hierzu.

Eine der wesentlichen Aufgaben in der Dialyse besteht im Entzug von Wasser aus dem Patientenblut. Da der Patient in der Regel nur noch wenig bis überhaupt nicht in der Lage ist, Wasser bzw. Urin auszuscheiden, werden während einer Behandlung bis zu 31 Wasser aus dem Patienten entfernt (Ultrafiltration). Der Zugang zu diesem Wasser ist nur über das Blut möglich. Wenn also Wasser im Blut fehlt, steigt die Dichte und Viskosität des Blutes an. Das Blut wird "dicker". Der Zusammenhang kann wie folgt erklärt werden:

Blut besteht im Wesentlichen aus Plasma und Blutzellen. Plasma wiederum setzt sich zusammen aus Wasser und darin gelösten Stoffen wie Elektrolyten, Proteinen und Stoffwechselprodukten. Bei Blutzellen spricht man von roten und weißen Blutkörperchen sowie von Blutplättchen, wobei die roten Blutkörperchen zahlenmäßig bei weitem überwiegen.

Der Hämatokrit (Hkt) ist der Anteil der zellulären Bestandteile am gesamten Blutvolumen. Aufgrund der Häufigkeit der roten Blutkörperchen wird oft vereinfacht der Anteil der roten Blutkörperchen am Blutvolumen herangezogen. Das relative Blutvolumen (RBV), das für die Regelung der Ultrafiltration in Dialysemaschinen verwendet wird, berechnet sich aus dem Hämatokrit zum Bezugszeitpunkt ($Hkt(t_0)$) und dem aktuellen Hämatokrit zu ($Hkt(t)$)

$$RBV = \frac{Hkt(t_0)}{Hkt(t)}$$

Die Dichte von Blutplasma (~1,03 g/cm³) unterscheidet sich nur gering von der Dichte der roten Blutkörperchen (~1,10 g/cm³). Dadurch ergibt sich bei einem sehr weiten Hämatokritbereich von 15% bis 60% ein Dichtespektrum von nur 1,04 - 1,07 g/cm³. Die Viskosität von Blut ändert sich im Gegensatz zur Dichte deutlich bei variierendem Hämatokrit, wie dies in nachstehender Tabelle dargestellt ist:

| Hämatokrit (Hkt) in % | Viskosität in cP |
|---|---|
| 15 | 2,05 |
| 35 | 2,89 |

(fortgesetzt)

| Hämatokrit (Hkt) in % | Viskosität in cP |
|---|---|
| 40 | 3,26 |
| 45 | 3,74 |
| 50 | 4,36 |
| 60 | 6,1 |

Der Anstieg der Dichte des Blutes ist - wie oben beschrieben - nicht besonders deutlich. Bestehende Messverfahren, welche diese Dichteunterschiede messen wollen, müssen entsprechend genau arbeiten können. Da in jedem Fall das Messmittel von außen durch eine Gefäßwandung hindurch messen muss, unterliegen alle bisherigen Messmethoden stark den Einflüssen von Materialeigenschaften und Messmittel-Ankopplung. Die Kosten solcher Systeme steigen mit steigender Qualität der Messstrecke (z. B. exakte Schlauchdurchmesser, Küvetten im Disposable etc.)

Existierende Blutvolumenmessgeräte können Ultraschall zur Messung des Hkt verwenden, bei optischen Verfahren wird mit einer Lichtquelle über Streuung bzw. Reflexion ein Maß "Lichtstörender Partikel" ermittelt.

Zur Durchführung und Überwachung der Ultrafiltration wird beispielsweise über ein Bilanziersystem gemäß der EP 0 867 195 B1 sichergestellt, dass die Menge an in den Dialysierfilter einfließendem Dialysat gleich der Menge des Dialysats ist, das aus dem Dialysierfilter wieder herausfließt. Über eine Ultrafiltrationspumpe kann dann kontrolliert eine zusätzliche Menge an Flüssigkeit aus dem Dialysierfilter entzogen werden. Diese Pumpe erzeugt einen entsprechenden Transmembrandruck, der Flüssigkeit über die Poren der Fasern des Dialysierfilters aus dem Blut des Patienten auf die Dialysatseite zieht, was fachüblich ultrafiltrieren genannt wird. Über die Gabe von Dilutionslösungen stromauf (sog. Prädilution) oder stromab (sog. Postdilution) des Filters kann die Clearance der Dialysebehandlung vergrößert werden, weil hierdurch der konvektive Stofftransport über die semipermeable Membran von der Blutseite zur Dialysatseite weiter erhöht wird. Der Patient kann so kontrolliert entwässert werden. Eine Vorgabe der Ultrafiltrationsrate und eines Ultrafiltrationszieles wird dabei durch den behandelnden Arzt vorgegeben. Diese bekannten Bilanziersysteme sind jedoch sehr aufwendig und auch teuer in der Herstellung.

Grundsätzlich ist bei Hämodialysemaschinen vorgesehen, das zu behandelnde Blut des Patienten im extrakorporalen Blutkreislauf mittels Schlauchpumpen zu fördern. Vereinzelt wird jedoch auch beschrieben, Zentrifugalpumpen in der Dialyse zu verwenden. Beispielsweise die US 6,439,845 betrifft eine Zentrifugalpumpe für ein Dialysiergerät, das tragbar und/oder implantierbar ist.

Darüber hinaus ist es aus dem Stand der Technik bekannt, Zentrifugalpumpen zur Messung der Viskosität von Flüssigkeiten, unter Anderem auch von Blut, zu verwenden.

Die EP 1 284 369 A1 bzw. die EP 1 284 370 A1 betrifft ein Verfahren und eine Pumpvorrichtung zum Erzeugen eines einstellbaren, im Wesentlichen konstanten Volumenstroms. Dabei wird durch eine Messung des Motorstroms die Viskosität des zu fördernden Fluids in einfacher Weise und online bestimmt. Hierzu wird zur Bestimmung der Viskosität eine feste Testdrehzahl des Rotors eingestellt und der Motorstrom bei dieser Testdrehzahl bestimmt. Mit dem bekannten anliegenden Motorstrom kann sodann die Viskosität des Fluids anhand von vorab erfolgten Eichmessungen und/oder Berechnungen ermittelt werden. Die Durchführung dieses Verfahrens wird im Zusammenhang mit Fotolacken oder für Polierzwecke verwendeten Suspensionen von feinen Festkörperteilchen in einer Flüssigkeit beschrieben.

Die EP 1 424 089 A2 offenbart eine Vorrichtung zur Regelung/Steuerung der Infusion einer Flüssigkeit in einem extrakorporalen Blutkreislauf. Die Vorrichtung umfasst dabei eine Regelungs-/Steuerungseinheit, die anhand von charakteristischen Messwerten, die mit der Blutkonzentration und/oder einer Filtrationseffizienz eines Filters korrelieren, eine Infusionssequenz bestimmt. Weiterhin regelt/steuert die Vorrichtung der EP 1 424 089 A2 die Infusion in Übereinstimmung mit der zuvor bestimmten Infusionssequenz.

Aus der EP 2 037 236 A2 ist weiter ein Verfahren zur Kalibrierung einer Durchflussmessung in einem Strömungssystem sowie ein Strömungssystem zur Durchführung dieses Verfahrens bekannt. Das zu fördernde Fluid wird mittels einer Rotationspumpe bzw. Zentrifugalpumpe durch das Strömungssystem befördert und die Durchflussmenge aus einem elektrischen Betriebsparameter der Pumpe bestimmt sowie zu einem vorgegebenen Zeit mit einem Kalibriersensor mittels Ultraschall die Durchflussmenge des Fluids in einer Kalibriermessung ermittelt und die Durchflussmessung anhand der Kalibriermessung neu kalibriert.

Aus der DE 102 24 750 A1 ist weiter bekannt, Hämodialysemaschinen mit in diese einsetzbaren, disposablen Kassettensystemen zu versehen, wobei diese Kassetten aus einem im Wesentlichen starren Kassettengrundkörper mit eingelassenen Kammern und Kanälen und einer diese abdeckenden Folie bestehen. Dabei sind in der Flüssigkeitsbehandlungsmaschine Aktoren und Sensoren zum Betrieb der Vorrichtung in der eingesetzten Kassette derart angeordnet, dass die Kassetten in verschiedenen Integrationsformen einsetzbar sind.

In der Praxis werden Zentrifugalpumpen insbesondere bei Herz-Lungenmaschinen eingesetzt. Eine derartige Herz-Lungenmaschine mit einer Zentrifugalpumpe ist z. B. in der EP 1 661 593 B1 offenbart.

Aus dem Stand der Technik gab es bereits des Weiteren den Vorschlag, die Viskosität des Blutes als Rückführgröße zur Regelung der Ultrafiltrationsrate bei Hämodialysebehandlungen zu nutzen (Greenwood, RN et al.; Serial blood water estimations and in-line blood viscometry: the continuous measurement of blood volume during dialysis procedures; in: Clinical Science, 1984, 66, pp. 575-583).

Es ist daher die Aufgabe der vorliegenden Erfindung, eine Vorrichtung zur Behandlung einer medizinischen Flüssigkeit in vorteilhafter Weise weiterzubilden, insbesondere dahingehend, dass diese einfach aufgebaut ist und zugleich eine hochgenaue Ermittlung von Parametern betreffend die Ultrafiltration ermöglicht.

Diese Aufgabe wird erfindungsgemäß gelöst durch eine Vorrichtung mit den Merkmalen des Anspruchs 1. Danach ist eine Vorrichtung zur Regelung wenigstens eines Filtrationswertes in einer Maschine zur Behandlung einer medizinischen Flüssigkeit, insbesondere einer Blutbehandlungsmaschine, vorgesehen, wobei die Viskosität der medizinischen Flüssigkeit mittels der Vorrichtung ermittelbar ist und wobei anhand der ermittelten Viskosität der wenigstens eine Filtrationswert regelbar ist, wobei die Vorrichtung wenigstens ein Zentrifugalpumpmittel aufweist und dass mittels des Zentrifugalpumpmittels die Viskosität der medizinischen Flüssigkeit mittelbar oder unmittelbar ermittelbar ist und wobei das Zentrifugalpumpmittel in wenigstens einem Fördermodus und in wenigstens einem Messmodus betreibbar ist und in dem Messmodus mit hinreichend geringer Drehzahl betrieben wird, so dass die medizinische Flüssigkeit nicht durch das Zentrifugalpumpmittel gefördert sondern in diesem zirkuliert wird, und wobei die Viskosität der medizinischen Flüssigkeit ohne zusätzliche Absperrvorrichtungen ermittelbar ist.

Ein Filtrationswert kann beispielsweise eine zu filtrierende Menge, ein Volumenstrom pro Zeiteinheit oder ein sonstiger im Zusammenhang mit der Filtration denkbarer Wert sein. Der Filtrationswert bezieht sich dabei insbesondere auf die in der Maschine zu behandelnde Flüssigkeit. Diese Flüssigkeit kann beispielsweise Blut sein.

Die Maschine zur Behandlung einer medizinischen Flüssigkeit ist dabei in vorteilhafter Ausgestaltung eine Blutbehandlungsmaschine. Denkbar ist ferner, dass die Vorrichtung in Zellseparatoren und/oder bei der Apherese verwendet wird.

Dadurch ergibt sich der Vorteil, dass die Regelung eines Filtrationswertes erfolgen kann, ohne dass beispielsweise zusätzliche Sensoren vorzusehen sind, da das Zentrifugalpumpmittel selbst zur Ermittlung der erforderlichen Regelgröße dient. Einfach ausgedrückt, werden Sensor und Pumpe nunmehr vorteilhafterweise zumindest funktional als ein Teil ausgeführt. Dabei ist darauf hinzuweisen, dass z. B. aus Sicherheitsgründen weiterhin zusätzliche Sensoren zur Ermittlung insbesondere der Flussrate vorgesehen sein können, dies ist jedoch nunmehr nicht zwingend erforderlich. Auch ist es nun möglich, vollständig auf kostenintensive Bilanzierungssysteme zu verzichten, da eine Ermittlung der entsprechenden Regelgröße und eine ausreichend genaue Flüssigkeitsförderung durch das eingesetzte Zentrifugalpumpmittel selbst geleistet werden kann. Auch auf einen Blutvolumenmonitor (BVM) kann verzichtet werden. Hierdurch kann eine signifikante Kostenreduktion erreicht werden.

Darüber hinaus ist eine wesentlich genauere Ermittlung einer Regelgröße zur Ermittlung eines Filtrationswertes im Vergleich zu den bisherigen Ansätzen möglich, da hierzu auf die Viskosität der Flüssigkeit abgestellt werden kann, was Vorteile z. B. im Vergleich zu einer Dichtemessung der Flüssigkeit bietet. Ein weiterer Vorteil ergibt sich daraus, dass nunmehr eine direkte Messung in der Flüssigkeit möglich ist, so dass Messfehler, die durch indirekte Messverfahren bedingt sind, nicht auftreten.

Des Weiteren kann vorgesehen sein, dass das Zentrifugalmittel Teil einer Zentrifugalpumpe oder eine Zentrifugalpumpe ist oder umfasst und/oder dass die medizinische Flüssigkeit mittels des Zentrifugalpumpmittels förderbar ist.

Ferner ist denkbar, dass das Zentrifugalpumpmittel in wenigstens einem Fördermodus und/oder wenigstens einem Messmodus betreibbar ist. Beispielsweise kann vorgesehen sein, dass das Zentrifugalpumpmittel bei normalem Betrieb im Fördermodus läuft, etwa bei der vorgegebenen Pumprate z. B. für den extrakorporalen Kreislauf. Periodisch kann sodann das Zentrifugalpumpmittel in einen Messmodus geschaltet werden, in dem mittelbar und/oder unmittelbar die Viskosität der medizinischen Flüssigkeit ermittelbar ist, so dass anhand der ermittelten Viskosität der wenigstens eine Filtrationswert geregelt werden kann, da durch die ermittelte Viskosität eine entsprechende Regelgröße zur Verfügung steht.

Ferner ist denkbar, dass die Viskosität der medizinischen Flüssigkeit im Fördermodus des Zentrifugalpumpmittels ermittelbar ist, wobei ein zusätzlicher Flussmesssensor vorhanden ist, der den Fluss der medizinischen Flüssigkeit ermittelt. Ein Verfahren hierzu offenbart die EP 1 284 369 A1 bzw. die EP 1 284 370 A1, auf die hier vollumfänglich verwiesen wird.

Außerdem kann vorgesehen sein, dass das Zentrifugalpumpmittel zumindest einen antreibenden ersten Teil und einen angetriebenen zweiten Teil aufweist. Denkbar ist, dass die Teile des Zentrifugalpumpmittels, also der antreibende erste Teil und der angetriebene zweite Teil derart relativ zueinander angeordnet sind, dass sie im zusammengesetzten Zustand pumpfähig sind, jedoch nach Ende insbesondere der Flüssigkeitsbehandlung voneinander getrennt werden können. Dies erleichtert insbesondere die Handhabung aber auch die Wartung des Zentrifugalpumpmittels. Weiter kann vorgesehen sein, dass mittels magnetischer Kopplung eine Drehmoment- und/oder Kraftübertragung vom antreibenden ersten Teil zum angetriebenen zweiten Teil erfolgt. Dadurch wird es möglich, eine berührungslose und damit verschleißarme Drehmoment- und/oder Kraftübertragung zu ermöglichen.

Bei dieser Ausführungsform gibt es keine Verluste in einer mechanischen Lagerung des Pumpmittels, so dass das Antriebsmoment des Pumpmittels ein Maß für die Viskosität der gepumpten Flüssigkeit ist.

Grundsätzlich ist denkbar, dass das antreibende erste Teil des Zentrifugalpumpmittels und das angetriebene zweite Teil des Zentrifugalpumpmittels auch auf andere Art und Weise als durch magnetische Kopplung berührungslos gekoppelt sind.

Darüber hinaus kann vorgesehen sein, dass die Viskosität der medizinischen Flüssigkeit anhand der Drehzahl und des Antriebsmoments des Zentrifugalpumpmittels mittelbar und/oder unmittelbar ermittelbar ist. Dies kann beispielsweise anhand des aufgenommenen Motorstromes erfolgen. Denkbar ist insbesondere, im Messmodus einen festen Motorstrom vorzugeben und die Drehzahl zu ermitteln und/oder eine feste Drehzahl vorzugeben und den hierfür benötigten Motorstrom zu ermitteln. Eine Durchführung und Auswertung kann beispielsweise mittels des Steuerungs- und/oder Regelungsmittels des Zentrifugalpumpmittels oder der Vorrichtung selbst erfolgen.

Des Weiteren kann vorgesehen sein, dass die Vorrichtung zumindest teilweise als Disposable ausgeführt ist bzw. austauschbare Komponenten aufweist, die als Disposable ausgeführt sind.

Insbesondere ist möglich, dass im Disposable wenigstens der angetriebene zweite Teil des Zentrifugalpumpmittels angeordnet ist und/oder dass das Disposable kassettenartig oder als Disposablekassette ausgebildet ist. Dies ist vorteilhaft möglich, da die Qualität des Disposables aufgrund der gewählten Messmethode keinen Einfluss auf die Messgröße selbst hat.

Es ist weiter beispielsweise auch nicht erforderlich, eine zusätzliche Ankopplung an das Disposable vorzunehmen. Dies ist insbesondere dann der Fall, wenn mittels magnetischer Kopplung eine Drehmoment- und/oder Kraftübertragung vom antreibenden ersten Teil zum angetriebenen zweiten Teil erfolgt. Dadurch ergibt sich der Vorteil, dass die notwendigen Parameter bereits über die berührungslose magnetische Ankopplung übertragen und hieraus ermittelt werden können.

Weiter ist möglich, dass die Maschine zur Behandlung einer medizinischen Flüssigkeit eine Hämodialysemaschine ist und/oder dass der Filtrationswert bzw. die Filtrationswerte der Ultrafiltrationsrate und/oder das Ultrafiltrationsziel ist bzw. sind. Denkbar ist insbesondere, dass die Ultrafiltrationsrate oder das Ultrafiltrationsziel mittels Eingabemitteln in die Vorrichtung eingebbar und/oder einspeicherbar sind. Dadurch ergibt sich der Vorteil, dass beispielsweise der behandelnde Arzt diese Werte für Ultrafiltrationsrate und/oder das Ultrafiltrationsziel vorab einfach eingeben kann.

Bevorzugt wird es insbesondere, wenn die medizinische Flüssigkeit Blut ist und dass anhand der ermittelten Viskosität der Hämatokritwert des Blutes ermittelbar ist. Hierdurch kann besonders vorteilhaft der Zustand des Blutes ermittelt werden. Weiter kann hieraus einfach und vorteilhaft ermittelt werden, wie viel Flüssigkeit bereits aus dem Blut entzogen wurde, so dass hieraus Rückschlüsse auf den Filtrationswert möglich sind.

Denkbar ist es insbesondere auch, dass die medizinische Flüssigkeit Dialysat ist, und dass anhand der ermittelten Viskosität besonders vorteilhaft der Zustand des Blutes ermittelt werden kann. Insbesondere kann hieraus einfach und vorteilhaft ermittelt werden, wie viel Flüssigkeit bereits aus dem Blut über die semipermeable Membran des Dialysefilters in das Dialysat transportiert wurde, so dass hieraus Rückschlüsse auf den Filtrationswert möglich sind. Voraussetzung für die Regelung der Filtration ist insbesondere, dass die Bestimmung der Viskosität der medizinischen Flüssigkeit flußabwärts bzw. stromabwärts des Dialysefilters erfolgt.

Denkbar ist es auch, dass die medizinische Flüssigkeit Filtrat ist, beispielsweise bei der Plasmaseparation, wobei in besonders vorteilhafter Weise die Viskosität des dort abfiltrierten Plasmas ermittelbar ist.

Ferner kann vorgesehen sein, dass die Vorrichtung wenigstens ein Steuerungs- und/oder Regelungsmittel aufweist und/oder wenigstens einen Anschluss aufweist, der mit wenigstens einem Steuerungs- und/oder Regelungsmittel verbindbar ist, wobei mittels des Steuerungs- und/oder Regelungsmittels mittelbar und/oder unmittelbar die Viskosität der medizinischen Flüssigkeit ermittelbar ist und/oder der wenigstens eine Filtrationswert regelbar ist.

Weiter ist denkbar, dass mittels des Steuerungs- und/oder Regelungsmittels das Zentrifugalpumpmittel zwischen Fördermodus und Messmodus umschaltbar ist und/oder dass mittels des Steuerungs- und/oder Regelungsmittels die Drehzahl des Zentrifugalpumpmittels regelbar ist.

Darüber hinaus kann vorgesehen sein, dass das Zentrifugalpumpmittel eingangsseitig und/oder ausgangsseitig wenigstens ein Verschlussmittel aufweist, mittels dessen das Zentrifugalpumpmittel eingangsseitig und/oder ausgangsseitig verschließbar ist. Vorgesehen sein kann in diesem Zusammenhang insbesondere, dass das Verschlussmittel eine Klemme oder ein Stößel ist, der den Zulauf eingangsseitig und/oder ausgangsseitig des Zentrifugalpumpmittels verschließen kann. Die Ansteuerung dieses Verschlussmittels kann beispielsweise durch das Steuerungs- und/oder Regelungsmittel erfolgen.

Es ist möglich, dass das Zentrifugalpumpmittel im Messmodus betreibbar ist, wenn wenigstens ein Verschlussmittel das Zentrifugalpumpmittel eingangsseitig und/oder ausgangsseitig verschließt, vorzugsweise, wenn beide Verschlussmittel das Zentrifugalpumpmittel eingangsseitig und ausgangsseitig verschließen. Denkbar ist, eine vollständige bzw. nahezu vollständige Rezirkulation über die Fördermittel des Zentrifugalpumpmittels wie das Rotorblatt einer Zentrifugalpumpe herbeizuführen. Die Viskosität der rezirkulierenden Flüssigkeit bestimmt die Drehzahl des Rotors der Zentrifugalpumpe bei einem bestimmten Antriebsmoment der Pumpe. Die Rotordrehzahl kann hierbei durch bekannte Messeinrichtungen gemessen werden (z. B. Hallsonde, Tachowelle). Die Rotordrehzahl ist näherungsweise der Betriebsspannung der Zentrifugalpumpe direkt proportional, weswegen die Betriebsspannung auch als ein Maß für die Rotordrehzahl herangezogen werden kann. Das Antriebsmoment ist näherungsweise dem Betriebsstrom der Zentrifugalpumpe direkt proportional, weswegen der Betriebsstrom auch als ein Maß für das Antriebsmoment herangezogen werden kann. Bei bekannter Rotordrehzahl und bekanntem Antriebsmoment der Zentrifugalpumpe kann auf die Viskosität der rezirkulierenden Flüssigkeit geschlossen werden. Die EP 1 284 369 A1 bzw. die EP 1 284 370 A1 und auch die EP 0 967 475 A1 offenbaren hierzu Verfahren. Da sich die Viskosität während des Betriebes der Maschine zur Behandlung einer medizinischen Flüssigkeit ändert bzw. ändern soll, wie dies insbesondere bei einer Blutbehandlungsmaschine und hier insbesondere bei einer Hämodialysemaschine der Fall ist, stellt die so ermittelte Viskosität eine Information über den Flüssigkeitsstatus der medizinischen Flüssigkeit und insbesondere des Blutes dar. Ist die aktuelle Viskosität ermittelt, kann mit einem neuen Offset-Wert der Viskosität eine genauere - korrigierte - Berechnung des Pumpen-Volumenstroms vorgenommen werden. Dadurch wird eine besonders vorteilhafte Regelung des wenigstens einen Filtrationswertes ermöglicht.

Gemäß der EP 0 967 475 A1 ist es möglich, zur Bestimmung der Viskosität der medizinischen Flüssigkeit, die von einem Zentrifugalpumpmittel gefördert wird, die Viskosität aus Messgrößen betreffend das Zentrifugalpumpmittel bzw. den Rotor des Zentrifugalpumpmittels zu bestimmen. Als Messgrößen können das Antriebsmoment bzw. der Antriebsstrom des Rotors und/oder die Rotordrehzahl gemessen werden. Aus den Werten für Antriebsmoment bzw. Antriebsstrom kann sodann die Viskosität der medizinischen Flüssigkeit bestimmt werden. Eine Drehzahl kann vorgegeben werden und die tatsächliche Drehzahl z. B. mittels eines Hallsensors erfasst werden.

Eine weitere Ausführungsform kann darin bestehen, dass stromabwärts oder stromaufwärts des Zentrifugalpumpmittels wenigstens ein Erfassungsmittel, insbesondere ein Flussmesssensor, zur mittelbaren und/oder unmittelbaren Erfassung und/oder Ermittlung des Volumenstromes der medizinischen Flüssigkeit angeordnet ist. So kann analog dem Verfahren gemäß der EP 1 284 369 A1 bzw. der EP 1 284 370 A1 eine Viskositätsmessung dann "online", also während des Förderbetriebes erfolgen.

Weiter kann vorgesehen sein, dass das Zentrifugalpumpmittel in einem Modus mit hinreichend geringer Drehzahl betreibbar ist, so dass die medizinische Flüssigkeit nicht durch das Zentrifugalpumpmittel förderbar, sondern im Zentrifugalpumpmittel zirkulierbar ist bzw. nicht durch das Zentrifugalpumpmittel gefördert sondern in diesem zirkuliert wird, wobei die hinreichend niedrige Drehzahl vorzugsweise wenige hundert Umdrehungen pro Minute, insbesondere 200 U/min oder auch weniger beträgt und wobei weiter vorzugsweise die Viskosität der medizinischen Flüssigkeit ohne zusätzliche Flussmesssensoren und/oder Absperrvorrichtungen ermittelbar ist. Es hat sich gezeigt, dass bei hinreichend niedrigen Drehzahlen des Zentrifugalpumpmittels, beispielsweise wenige hundert Umdrehungen pro Minute, insbesondere 200 U/min oder auch weniger, keine Flüssigkeit durch das Zentrifugalpumpmittel gefördert wird, sondern ausschließlich im Zentrifugalpumpmittel zirkuliert. In dieser Betriebsart kann dann analog des weiter oben dargelegten Verfahrens die Viskosität bestimmt werden, ohne zusätzliche Flussmesssensoren oder Absperrvorrichtungen. Es kann also in einem Fall, in dem die Drehzahl gering genug ist, so dass z. B. kein Blut, Dialysat oder Filtrat durch das Zentrifugalpumpmittel gefördert wird, eine Viskositätsmessung sowohl ohne Klemme als auch ohne eine Flussmessung erfolgen.

Vorteilhaft ist denkbar, dass der Volumenstrom der medizinischen Flüssigkeit konstant ist, und/oder weiter vorteilhaft, dass das Zentrifugalpumpmittel mit einem Wirkungsgrad betrieben wird, der kleiner ist als die Hälfte des maximalen Wirkungsgrads, vorzugsweise mit höchstens 20% des maximalen Wirkungsgrads des Zentrifugalpumpmittels.

Des Weiteren betrifft die vorliegende Erfindung eine Hämodialysemaschine mit den Merkmalen des Anspruchs 12. Danach ist vorgesehen, dass eine Hämodialysemaschine wenigstens eine Vorrichtung nach einem der Ansprüche 1 bis 11 aufweist.

Darüber hinaus kann vorgesehen sein, dass die Hämodialysemaschine eine Aufnahme aufweist, in die ein Disposable einsetzbar ist, das zumindest Bestandteile des Zentrifugalpumpmittels aufweist, mittels dessen mittelbar und/oder unmittelbar die Viskosität der medizinischen Flüssigkeit ermittelbar ist, wobei vorzugsweise der antreibende erste Teil des Zentrifugalpumpmittels in der Hämodialysemaschine angeordnet ist.

Außerdem betrifft die vorliegende Erfindung ein Verfahren zur Regelung eines Filtrationswertes mit den Merkmalen des Anspruchs 15. Danach ist vorgesehen, dass ein Verfahren zur Regelung wenigstens eines Filtrationswertes in einer Maschine zur Behandlung einer medizinischen Flüssigkeit, insbesondere einer Blutbehandlungsmaschine, mit wenigstens einer Vorrichtung nach einem der Ansprüche 1 bis 11 derart durchgeführt wird, dass mittels des Zentrifugalpumpmittels mittelbar und/oder unmittelbar die Viskosität der medizinischen Flüssigkeit ermittelt wird und dass anhand der ermittelten Viskosität der wenigstens eine Filtrationswert geregelt wird.

Weiterhin kann vorgesehen sein, dass das Zentrifugalpumpmittel in wenigstens einem Fördermodus und/oder wenigstens einem Messmodus betrieben wird und/oder dass die Viskosität der medizinischen Flüssigkeit anhand von Drehzahl und Antriebsmoment des Zentrifugalpumpmittels mittelbar und/oder unmittelbar ermittelt wird.

Darüber hinaus kann vorgesehen sein, dass das Verfahren mittels einer Vorrichtung nach einem der Ansprüche 1 bis 11 und/oder eine Hämodialysemaschine nach einem der Ansprüche 12 bis 14 durchgeführt wird.

Weitere Einzelheiten und Vorteile der Erfindung sollen nun anhand eines in der Zeichnung dargestellten Ausführungsbeispiels näher erläutert werden.

Es zeigt:

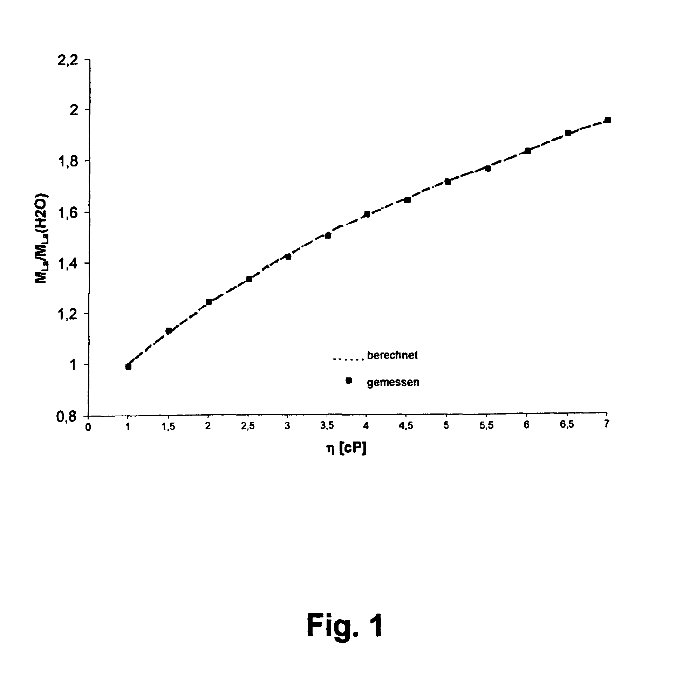

Figur 1: den Zusammenhang zwischen der Viskosität und dem hydraulischen Moment bei einer Rotordrehzahl einer Zentrifugalpumpe von 4000 U/min.

Ein Ausführungsbeispiel der Erfindung, mittels dessen besonders vorteilhaft eine Umsetzung der Erfindung erfolgen kann, wird dadurch bereitgestellt, dass anstelle einer okkludierenden Schlauchrollenpumpe für den Bluttransport im extrakorporalen Blutkreislauf bei einer Hämodialysemaschine eine Zentrifugalpumpe eingesetzt wird. Diese Zentrifugalpumpe wird dabei einerseits in einem Fördermodus zur Förderung des Blutes benutzt und andererseits in einem Messmodus zur Bestimmung der Viskosität des Blutes. Dieser durch die Messung ermittelte Wert für die Viskosität des Blutes wird weiter dazu benutzt, den Hämatokritgehalt des Blutes zu bestimmen. Dabei kann anhand des zeitlichen Verlaufs des Hämatokrits das relative Blutvolumen bestimmt werden. Hierdurch wird eine Regelgröße für die Ultrafiltration erzeugt. Die erfindungsgemäße Vorrichtung ist bei diesem Ausführungsbeispiel integraler Bestandteil der Hämodialysemaschine.

Die vom Arzt vorgegebene Ultrafiltrationsrate und das Ultrafiltrationsziel, also die Gesamtmenge an entzogenem Wasser wird erfindungsgemäß derart geregelt, dass nicht mehr die absolute Menge der Flüssigkeit, die dem Blut entzogen wird, überwacht wird, sondern die Konzentration des Wassers im Blut über dessen Viskosität bzw. Hämatokritgehalt und deren zeitlichen Verlauf. Zur Bestimmung der Viskosität wird die Zentrifugalpumpe regelmäßig, also in periodisch wiederkehrenden Abständen, vom Blutfördermodus in einen Messmodus umgeschaltet.

Die Bestimmung der Viskosität kann bei bekanntem Flüssigkeitsfluss auch während des Förderbetriebs ermittelt werden. Eine Unterbrechung der Flüssigkeitsförderung ist dann nicht mehr notwendig.

Zum Betrieb im Messmodus werden hierzu die Pumpenein- und -ausgänge verschlossen, wozu entsprechende Verschlussmittel vorgesehen sind. Dies können beispielsweise Klemmen oder Stößel sein, die die Zuführ- und Abführleitung durch Zusammendrücken verschließen. Das in der Pumpkammer befindliche Blut wird sodann rezirkuliert. Die Viskosität des rezirkulierenden Blutes bestimmt die Drehzahl des Rotors der Zentrifugalpumpe bei einem bestimmten Antriebsmoment der Pumpe. Die Rotordrehzahl kann hierbei durch bekannte Messeinrichtungen gemessen werden (z. B. Hallsonde, Tachowelle). Die Rotordrehzahl ist näherungsweise der Betriebsspannung der Zentrifugalpumpe direkt proportional, weswegen die Betriebsspannung auch als ein Maß für die Rotordrehzahl herangezogen werden kann. Das Antriebsmoment ist näherungsweise dem Betriebsstrom der Zentrifugalpumpe direkt proportional, weswegen der Betriebsstrom auch als ein Maß für das Antriebsmoment herangezogen werden kann. Bei bekannter Rotordrehzahl und bekanntem Antriebsmoment der Zentrifugalpumpe kann auf die Viskosität der rezirkulierenden Flüssigkeit geschlossen werden. Die EP 1 284 369 A1 bzw. die EP 1 284 370 A1 offenbart hierzu ein Verfahren. Da sich die Viskosität während des Betriebes der Maschine zur Behandlung einer medizinischen Flüssigkeit ändert bzw. ändern soll, wie dies insbesondere bei einer Blutbehandlungsmaschine und hier insbesondere bei einer Hämodialysemaschine der Fall ist, stellt die so ermittelte Viskosität eine Information über den Flüssigkeitsstatus des Blutes dar. Ist die aktuelle Viskosität ermittelt, kann mit einem neuen Offset-Wert der Viskosität eine genauere - korrigierte - Berechnung des Pumpen-Volumenstroms vorgenommen werden. Dadurch wird eine besonders vorteilhafte Regelung des wenigstens einen Filtrationswertes, z. B. für die vom Arzt vorgegebene Ultrafiltrationsrate und des vorgegebenen Ultrafiltrationszieles, vorgenommen.

Dieser Abgleich und die darauf aufbauende Regelung wird vorzugsweise durch die Steuerungs- und/oder Regelungsmittel der Hämodialysemaschine durchgeführt. Hierzu können gesonderte Steuerungs- und/oder Regelungseinheiten in der Hämodialysemaschine vorgesehen sein, genauso gut ist es jedoch ebenso möglich, dass die Steuerung und/oder Regelung durch die zentrale Steuerungs- und Regelungseinheit der Hämodialysemaschine erfolgt.

Vorteilhafterweise sind dabei redundante Steuerungs- und/oder Regelungsmittel vorgesehen.

Zusätzliche Sensoren zur Ermittlung der Viskosität des Blutes sind nicht erforderlich, wenn die Ermittlung der Viskosität in einem Messmodul stattfindet, bei dem die Aus- und/oder Eingänge des Zentrifugalpumpmittels verschlossen werden. Findet die Ermittlung der Viskosität während der Förderung der medizinischen Flüssigkeit statt, ist zumindest ein zusätzlicher Flussmesssensor zur Ermittlung des Flüssigkeitsflusses notwendig.

In besonders vorteilhafter Ausgestaltung ist vorgesehen, dass die Zentrifugalpumpe einen antreibenden ersten Teil und einen angetriebenen zweiten Teil aufweist, die magnetisch berührungslos miteinander gekoppelt sind. Die Kraftübertragung bzw. Drehmomentübertragung vom antreibenden ersten Teil zum angetriebenen zweiten Teil der Zentrifugalpumpe erfolgt somit mittels magnetischer Kopplung. Besonders vorteilhaft in diesem Zusammenhang ist es, wenn der angetriebene zweite Teil des Zentrifugalpumpmittels, also beispielsweise der Pumpenrotor der Zentrifugalpumpe sowie die entsprechende Pumpkammer mit Zulauf und Ablauf Bestandteil eines kassettenartig ausgebildeten Disposables ist.

Ein derartiges kassettenartiges Disposable, das analog einer Kassette gemäß der DE 102 24 750 A1 ausgeführt sein kann, kann in eine entsprechende Aufnahme der Hämodialysemaschine eingesetzt werden, wobei durch entsprechende Konnektoren ein einfaches und eindeutiges Einsetzen vorgegeben sein kann. Dabei kann insbesondere der antreibende erste Teil der Zentrifugalpumpe in der Hämodialysemaschine selbst angeordnet sein, und zwar im Bereich der Aufnahme für die Kassette. Des Weiteren ist möglich, dass die Verschlussmittel für den Zulauf und den Ablauf der Zentrifugalpumpe bzw. zur Zentrifugalpumpenkammer durch Stößel ausgebildet sind, die maschinenseitig angeordnet sind. Diese Stößel können beispielsweise kanalartig ausgebildete Zuläufe und Abläufe zu der Zentrifugalpumpenkammer zusammendrücken.

Die Einzelheiten der Ermittlung der Parameter zur Regelung der Ultrafiltration sollen nachfolgend theoretisch dargestellt werden:

Die eingangs angeführte Tabelle 1 zeigt, dass sich die Viskosität bei unterschiedlichen Hämatokrit-Werten viel deutlicher ändert als die Dichte. Erfindungsgemäß erfolgt die Regelung der Ultrafiltrationsrate bzw. der Parameter für die Ultrafiltration anhand der Messung der Viskosität des Blutes. Diese Messung findet direkt im Blut statt und ist nicht von der Disposablequalität abhängig. Zur Messung wird eine magnetisch gelagerte Disposable-Zentrifugalpumpe verwendet. Die Pumpe fördert das Blut durch den Dialysator. Gelegentliches Verschließen der Pumpenein- und -ausgänge führt zu einer 100%igen Rezirkulation über das Rotorblatt. Die Viskosität der rezirkulierenden Flüssigkeit bestimmt die Drehzahl des Rotors der Zentrifugalpumpe bei einem bestimmten Antriebsmoment der Pumpe. Die Rotordrehzahl kann hierbei durch bekannte Messeinrichtungen gemessen werden (z. B. Hallsonde, Tachowelle). Die Rotordrehzahl ist näherungsweise der Betriebsspannung der Zentrifugalpumpe direkt proportional, weswegen die Betriebsspannung auch als ein Maß für die Rotordrehzahl herangezogen werden kann. Das Antriebsmoment ist näherungsweise dem Betriebsstrom der Zentrifugalpumpe direkt proportional, weswegen der Betriebsstrom auch als ein Maß für das Antriebsmoment herangezogen werden kann. Bei bekannter Rotordrehzahl und bekanntem Antriebsmoment der Zentrifugalpumpe kann auf die Viskosität der rezirkulierenden Flüssigkeit geschlossen werden. Die EP 1 284 369 A1 bzw. die EP 1 284 370 A1 offenbart hierzu ein Verfahren. Da sich die Viskosität während des Betriebes der Maschine zur Behandlung einer medizinischen Flüssigkeit ändert bzw. ändern soll, wie dies insbesondere bei einer Blutbehandlungsmaschine und hier insbesondere bei einer Hämodialysemaschine der Fall ist, stellt die so ermittelte Viskosität eine Information über den Flüssigkeitsstatus der medizinischen Flüssigkeit und insbesondere des Blutes dar.

Ist die aktuelle Viskosität ermittelt, kann mit einem neuen Offset-Wert der Viskosität eine genauere - korrigierte - Berechnung des Pumpen-Volumenstroms vorgenommen werden.

Im folgenden sind die Zusammenhänge für die Ermittlung der Viskosität des Blutes nochmals dargestellt:

Brookshier und Tarbell beschreiben in Biorheology (Band 28: p. 569-587, Pergamon Press plc., USA 1992) die Abhängigkeit der Viskosität des Blutes vom Hämatokrit: $\eta=1{,}4175+5{,}878\cdot Hkt-15{,}98\cdot Hkt^2+31{,}964\cdot Hkt^3$, mit Hkt in %/100 und $\eta$ in cP.

Das hydraulische Moment $M_L$ und damit auch die Hydraulische Leistung einer Zentrifugalpumpe $P_L=M_L\omega$ sind Größen, die von der Dichte $\rho$ und der Viskosität $v$ des Fluids innerhalb der Pumpe abhängen.

Dabei besteht weiter zwischen Dichte und Volumenstrom ein linearer Zusammenhang. Gleiches gilt für den volumenstrom-unabhängigen Anteil des hydraulischen Moments, der ebenfalls eine lineare Abhängigkeit von der Dichte aufweist. Ferner besteht eine nichtlineare Abhängigkeit des volumenstrom-unabhängigen Anteils des hydraulischen Moments von der Viskosität. Da die Zentrifugalpumpe magnetisch gelagert ist und hierdurch keinerlei mechanische Reibungsverluste auftreten, ist dies hauptsächlich auf die hydraulischen Reibungsverluste zwischen Laufrad und Gehäuse zurückzuführen.

Bei einer Blockade des Durchflusses durch die Pumpe bei einer beliebigen Drehzahl ergibt sich für das hydraulische Moment folgender Zusammenhang:

$$M_L\left(Q=0\right)=\rho\cdot\left(k_2\left(v\right)\cdot\omega^2+k_1\left(v\right)\cdot\omega\right).$$

Dabei weisen die Pumpenparameter k eine quadratische Abhängigkeit von der Viskosität auf. Die Pumpe arbeitet unter diesen vorgenannten Randbedingungen vergleichbar einem Rotationsviskosimeter.

Wenn eine feste Drehzahl verwendet wird, ist es möglich, den Rechenaufwand deutlich zu reduzieren. Die Viskosität kann sodann folgendermaßen berechnet werden:

$$\eta=k_2 M_{L0}^{\;2}+k_1 M_{L0}+k_0$$

Dieser Zusammenhang wird in Figur bei einer Rotordrehzahl von 4000 U/min dargestellt.

Das Moment kann über die Stromaufnahme der Zentrifugalpumpe ermittelt werden. Mit Kenntnis dieser Größe erhält man die Viskosität und kann daraus den Hämatokrit berechnen. Mit dem Hämatokrit wiederum berechnet man das relative Blutvolumen und hat somit die Rückführgröße für die Regelung der Ultrafiltration.

Es sei an dieser Stelle auch darauf hingewiesen, dass das kurzzeitige Verschließen beider Pumpenseiten zu keiner Schädigung des Blutes führt.

## Patentansprüche

1. Vorrichtung zur Regelung wenigstens eines Filtrationswertes in einer Maschine zur Behandlung einer medizinischen Flüssigkeit, insbesondere einer Blutbehandlungsmaschine, wobei die Viskosität der medizinischen Flüssigkeit mittels der Vorrichtung ermittelbar ist und wobei anhand der ermittelten Viskosität der wenigstens eine Filtrationswert regelbar ist, **dadurch gekennzeichnet,** **dass** die Vorrichtung wenigstens ein Zentrifugalpumpmittel aufweist und dass mittels des Zentrifugalpumpmittels die Viskosität der medizinischen Flüssigkeit mittelbar oder unmittelbar ermittelbar ist und wobei das Zentrifugalpumpmittel in wenigstens einem Fördermodus und in wenigstens einem Messmodus betreibbar ist und in dem Messmodus mit hinreichend geringer Drehzahl betrieben wird, so dass die medizinische Flüssigkeit nicht durch das Zentrifugalpumpmittel gefördert sondern in diesem zirkuliert wird, und wobei die Viskosität der medizinischen Flüssigkeit ohne zusätzliche Absperrvorrichtungen ermittelbar ist.

2. Vorrichtung nach Anspruch 1, **dadurch gekennzeichnet, dass** die Viskosität der medizinischen Flüssigkeit ohne zusätzliche Flussmesssensoren ermittelbar ist.

3. Vorrichtung nach Anspruch 1 oder 2, **dadurch gekennzeichnet, dass** die Viskosität der medizinischen Flüssigkeit anhand von Drehzahl und Antriebsmoment des Zentrifugalpumpmittels mittelbar oder unmittelbar ermittelbar ist.

4. Vorrichtung nach einem der vorhergehenden Ansprüche, **dadurch gekennzeichnet, dass** die Vorrichtung zumindest teilweise als Disposable ausgeführt ist oder austauschbare Komponenten aufweist, die als Disposable ausgeführt sind.

5. Vorrichtung nach Anspruch 4, **dadurch gekennzeichnet, dass** im Disposable wenigstens ein angetriebener Teil des Zentrifugalpumpmittels angeordnet ist.

6. Vorrichtung nach Anspruch 4 oder 5, **dadurch gekennzeichnet, dass** das Disposable kassettenartig oder als Disposablekassette ausgebildet ist.

7. Vorrichtung nach einem der vorhergehenden Ansprüche, **dadurch gekennzeichnet, dass** die Maschine zur Behandlung einer medizinischen Flüssigkeit eine Hämodialysemaschine ist.

8. Vorrichtung nach einem der vorhergehenden Ansprüche, **dadurch gekennzeichnet, dass** der Filtrationswert bzw. die Filtrationswerte die Ultrafiltrationsrate und/oder das Ultrafiltrationsziel ist bzw. sind.

9. Vorrichtung nach einem der vorhergehenden Ansprüche, **dadurch gekennzeichnet, dass** die medizinische Flüssigkeit Blut ist und dass anhand der ermittelten Viskosität der Hämatokritwert des Blutes ermittelbar ist oder dass die medizinische Flüssigkeit Dialysat ist und dass anhand der ermittelten Viskosität der Zustand des Blutes ermittelbar ist oder dass die medizinische Flüssigkeit Filtrat ist, beispielsweise bei der Plasmaseparation, wobei in besonders vorteilhafter Weise die Viskosität des dort abfiltrierten Plasmas ermittelbar ist.

10. Vorrichtung nach einem der vorhergehenden Ansprüche, **dadurch gekennzeichnet, dass** die Vorrichtung wenigstens ein Steuerungs- und/oder Regelungsmittel aufweist und/oder wenigstens einen Anschluss aufweist, der mit wenigstens einem Steuerungs- und/oder Regelungsmittel verbindbar ist, wobei mittels des Steuerungs- und/oder Regelungsmittels mittelbar oder unmittelbar die Viskosität der medizinischen Flüssigkeit ermittelbar ist und der wenigstens eine Filtrationswert regelbar ist.

11. Vorrichtung nach Anspruch 10, **dadurch gekennzeichnet, dass** mittels des Steuerungs- und/oder Regelungsmittels das Zentrifugalpumpmittel zwischen Fördermodus und Messmodus umschaltbar ist oder dass mittels des Steuerungs- und/oder Regelungsmittels die Drehzahl des Zentrifugalpumpmittels regelbar ist.

12. Hämodialysemaschine mit wenigstens einer Vorrichtung nach einem der vorhergehenden Ansprüche.

13. Hämodialysemaschine nach Anspruch 12, **dadurch gekennzeichnet, dass** die Hämodialysemaschine eine Aufnahme aufweist, in die ein Disposable einsetzbar ist, das zumindest Bestandteile des Zentrifugalpumpmittels aufweist, mittels dessen mittelbar oder unmittelbar die Viskosität der medizinischen Flüssigkeit ermittelbar ist.

14. Hämodialysemaschine nach Anspruch 13, **dadurch gekennzeichnet, dass** ein antreibender Teil des Zentrifugalpumpmittels in der Hämodialysemaschine angeordnet ist.

15. Verfahren zur Regelung wenigstens eines Filtrationswerts in einer Maschine zur Behandlung einer medizinischen Flüssigkeit, insbesondere einer Blutbehandlungsmaschine, wobei die Maschine mit wenigstens einer Vorrichtung nach einem der Ansprüche 1 bis 11 ausgeführt ist,
**dadurch gekennzeichnet,**
**dass** mittels des Zentrifugalpumpmittels mittelbar und/oder unmittelbar die Viskosität der medizinischen Flüssigkeit ermittelt wird und dass anhand der ermittelten Viskosität der wenigstens eine Filtrationswert geregelt wird.

**Claims**

1. An apparatus for the regulation of at least one filtration value in a machine for the treatment of a medical fluid, in particular in a blood treatment machine, wherein the viscosity of the medical fluid can be determined by means of the apparatus and wherein the at least one filtration value can be regulated with reference to the viscosity determined,
**characterized in that**
the apparatus has at least one centrifugal pumping means; and **in that** the viscosity of the medical fluid can be determined indirectly or directly by means of the centrifugal pumping means and wherein the centrifugal pumping means can be operated in at least one conveying mode and at least one measurement mode and can be operated in the measurement mode with a sufficiently slow speed so that the medical fluid is not conveyed by the centrifugal pumping means, but is circulated in the centrifugal pumping means, and wherein the viscosity of the medical fluid can be determined without additional blocking apparatus.

2. An apparatus in accordance with claim 1, **characterized in that** the viscosity of the medical fluid can be determined without additional flow measurement sensors.

3. An apparatus in accordance with either of claims 1 or 2, **characterized in that** the viscosity of the medical fluid can be determined indirectly or directly with reference to the speed and driving torque of the centrifugal pumping means.

4. An apparatus in accordance with one of the preceding claims, **characterized in that** the apparatus is made at least partly as a disposable or has replaceable components which are made as disposables.

5. An apparatus in accordance with claim 4, **characterized in that** at least one driven part of the centrifugal pumping means is arranged in the disposable.

6. An apparatus in accordance with claim 4 or 5, **characterized in that** the disposable is made in a cassette-like manner or as a disposable cassette.

7. An apparatus in accordance with one of the preceding claims, charaterizd in that the machine for the treatment of a medical fluid is a hemodialysis machine.

8. An apparatus in accordance with one of the preceding claims, **characterized in that** the filtration value or the filtration values is or are the ultrafiltration rate and/or the ultrafiltration target.

9. An apparatus in accordance with one of the preceding claims, **characterized in that** the medical fluid is blood; and **in that** the hematocrit value of the blood can be determined with reference to the determined viscosity; or **in that** the medical fluid is dialysate; and **in that** the state of the blood can be determined with reference to the determined viscosity; or **in that** the medical fluid is filtrate, for example in the plasma separation, with the viscosity of the plasma filtered there being able to be determined in a particularly advantageous manner.

10. An apparatus in accordance with one of the preceding claims, **characterized in that** the apparatus has at least one control and/or regulation means and/or has at least one connection which can be connected to at least one control and/or regulation means, with the viscosity of the medical fluid being able to be determined and/or the at least one filtration value being able to be regulated indirectly or directly by means of the control and/or regulation means.

11. An apparatus in accordance with claim 10, **characterized in that** the centrifugal pumping means can be switched over between the conveying mode and the measurement mode by means of the control and/or regulation means; or **in that** the speed of the centrifugal pumping means can be regulated by means of the control and/or regulation means.

12. A hemodialysis machine comprising at least one apparatus in accordance with one of the preceding claims.

13. A hemodialysis machine in accordance with claim 12, **characterized in that** the hemodialysis machine has a mount into which a disposable can be inserted that has at least elements of the centrifugal pumping means by means of which the viscosity of the medical fluid can be determined indirectly or directly.

14. A hemodialysis machine in accordance with claim 13, **characterized in that** a driving part of the centrifugal pumping means is arranged in the hemodialysis machine.

15. A method for the regulation of at least one filtration value in a machine for the treatment of a medical fluid, in particular in a blood treatment machine, with the machine being configured with at least one apparatus in accordance with one of the claims 1 to 11, **characterized in that**
the viscosity of the medical fluid is determined indirectly and/or directly by means of the centrifugal pumping means; and **in that** the at least one filtration value is regulated with reference to the viscosity determined.

**Revendications**

1. Dispositif de régulation d'au moins une valeur de filtration dans une machine de traitement d'un liquide médical, en particulier une machine de traitement du sang, la viscosité du liquide médical pouvant être déterminée au moyen du dispositif et l'au moins une valeur de filtration pouvant être régulée à partir de la viscosité déterminée,
**caractérisé en ce que**
le dispositif comporte au moins un moyen de pompage centrifuge et **en ce que** le moyen de pompage centrifuge permet de déterminer la viscosité du liquide médical directement ou indirectement et le moyen de pompage centrifuge pouvant fonctionner dans au moins un mode de transport et au moins un mode de mesure et fonctionnant dans le mode de mesure avec une vitesse de rotation suffisamment faible, de telle sorte que le liquide médical n'est pas transporté à travers le moyen de pompage centrifuge mais qu'il circule dans celui-ci, et la viscosité du liquide médical pouvant être déterminée sans dispositif de blocage supplémentaire.

2. Dispositif selon la revendication 1, **caractérisé en ce que** la viscosité du liquide médical peut être déterminée sans capteurs de mesure du flux supplémentaires.

3. Dispositif selon la revendication 1 ou 2, **caractérisé en ce que** la viscosité du liquide médical peut être déterminée directement ou indirectement à partir de la vitesse de rotation et du couple d'entraînement du moyen de pompage centrifuge.

4. Dispositif selon l'une des revendications précédentes, **caractérisé en ce que** le dispositif est réalisé au moins en partie en tant qu'élément jetable ou comporte des composants remplaçables qui sont réalisés en tant qu'éléments jetables.

**5.** Dispositif selon la revendication 4, **caractérisé en ce qu'**au moins une partie entraînée du moyen de pompage centrifuge est disposée dans l'élément jetable.

**6.** Dispositif selon la revendication 4 ou 5, **caractérisé en ce que** l'élément jetable est réalisé à la manière d'une cassette ou en tant que cassette jetable.

**7.** Dispositif selon l'une des revendications précédentes, **caractérisé en ce que** la machine de traitement d'un liquide médical est un hémodialyseur.

**8.** Dispositif selon l'une des revendications précédentes, **caractérisé en ce que** la valeur de filtration ou les valeurs de filtration est/sont la vitesse d'ultrafiltration et/ou l'objectif d'ultrafiltration.

**9.** Dispositif selon l'une des revendications précédentes, **caractérisé en ce que** le liquide médical est du sang et qu'à partir de la viscosité déterminée, le taux d'hématocrite du sang peut être déterminé ou **en ce que** le liquide médical est du dialysat et qu'à partir de la viscosité déterminée, l'état du sang peut être déterminé ou **en ce que** le liquide médical est du filtrat, par exemple lors de la séparation du plasma, la viscosité du plasma alors filtré pouvant être déterminée de manière particulièrement avantageuse.

**10.** Dispositif selon l'une des revendications précédentes, **caractérisé en ce que** le dispositif comporte au moins un moyen de commande et/ou de régulation et/ou au moins un raccordement qui peut être relié à au moins un moyen de commande et/ou de régulation, le moyen de commande et/ou de régulation permettant de déterminer la viscosité du liquide médical directement ou indirectement et de réguler l'au moins une valeur de filtration.

**11.** Dispositif selon la revendication 10, **caractérisé en ce que** le moyen de commande et/ou de régulation permet de commuter le moyen de pompage centrifuge entre un mode de transport et un mode de mesure ou **en ce que** le moyen de commande et/ou de régulation permet de réguler la vitesse de rotation du moyen de pompage centrifuge.

**12.** Hémodialyseur comprenant au moins un dispositif selon l'une des revendications précédentes.

**13.** Hémodialyseur selon la revendication 12, **caractérisé en ce que** l'hémodialyseur comporte un logement dans lequel un élément jetable peut être inséré, qui comporte au moins des constituants du moyen de pompage centrifuge, au moyen duquel la viscosité du liquide médical peut être déterminée directement ou indirectement.

**14.** Hémodialyseur selon la revendication 13, **caractérisé en ce qu'**une partie d'entraînement du moyen de pompage centrifuge est disposée dans l'hémodialyseur.

**15.** Procédé de régulation d'au moins une valeur de filtration dans une machine de traitement d'un liquide médical, en particulier une machine de traitement du sang, la machine étant réalisée avec au moins un dispositif selon l'une des revendications 1 à 11, **caractérisé en ce que**
le moyen de pompage centrifuge permet de déterminer directement et/ou indirectement la viscosité du liquide médical et **en ce qu'**à partir de la viscosité déterminée, l'au moins une valeur de filtration est régulée.

**Fig. 1**

**EP 2 547 376 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0867195 B1
- US 6439845 B
- EP 1284369 A1
- EP 1284370 A1
- EP 1424089 A2
- EP 2037236 A2
- DE 10224750 A1
- EP 1661593 B1
- EP 0967475 A1

**In der Beschreibung aufgeführte Nicht-Patentliteratur**

- **GREENWOOD, RN et al.** Serial blood water estimations and in-line blood viscometry: the continuous measurement of blood volume during dialysis procedures. *Clinical Science,* 1984, vol. 66, 575-583
- Biorheology. Pergamon Press plc, 1992, vol. 28, 569-587